# EUROPÄISCHE PATENTANMELDUNG

(11) **EP 1 672 035 A2**
(43) Veröffentlichungstag der Anmeldung: **21.06.2006**
(21) Anmeldenummer: 05021120.0
(22) Anmeldetag: 28.09.2005
(51) Int. Cl.: C09C 1/00

(54) **Effektpigmente auf Basis dünner SiO2-Plättchen**

(30) Priorität: 08.10.2004 DE 102004049203
(71) Anmelder: Merck Patent GmbH, 64293 Darmstadt (DE)
(72) Erfinder: Pfaff, Gerhard, Dr., 64839 Münster (DE); Warthe, Doreen, 64347 Griesheim (DE); Dietz, Johann, Dr., 63218 Dietzenbach (DE); Foerderer, Cornelia, 64646 Heppenheim (DE)

(57) **Zusammenfassung**

Die vorliegende Erfindung betrifft Effektpigmente mit verbesserten optischen Eigenschaften auf der Basis von mit einer oder mehreren Schichten beschichteten SiO₂-Plättchen, wobei die SiO₂-Plättchen eine Dicke von 50 nm bis 150 nm aufweisen, Verfahren zu ihrer Herstellung sowie die Verwendung dieser Pigmente in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten.

## Beschreibung

Die vorliegende Erfindung betrifft Effektpigmente mit verbesserten optischen Eigenschaften auf der Basis von mit einer oder mehreren Schichten beschichteten SiO₂-Plättchen, wobei die SiO₂-Plättchen eine Dicke von 50 nm bis 150 nm aufweisen, Verfahren zu ihrer Herstellung sowie die Verwendung dieser Pigmente in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten.

Der Einsatz von Glanz- oder Effektpigmenten ist weit verbreitet. Bei Automobillacken, dekorativen Beschichtungen aller Art sowie bei der Einfärbung von Kunststoffen, bei Anstrich- und Druckfarben, insbesondere Farben für den Sicherheitsdruck, sowie bei Anwendungen in der dekorativen Kosmetik sind derartige Pigmente unverzichtbar geworden. In der sie umgebenden Matrix richten sich diese Pigmente idealerweise parallel zur Oberfläche der Beschichtung aus und entfalten durch ein kompliziertes Zusammenspiel von Interferenz, Reflexion und Absorption des auffallenden Lichtes ihre optische Wirkung. Dabei stehen für die verschiedenen Anwendungsfälle eine brillante Farbgebung, Wechsel zwischen verschiedenen Farben in Abhängigkeit vom Betrachtungswinkel, so genannte Farbflops, oder wechselnde Helligkeitseindrücke im Mittelpunkt des Interesses.

Die Herstellung solcher Pigmente erfolgt in der Regel durch Beschichtung von plättchenförmigen metallischen oder nichtmetallischen Substraten mit Metalloxid- oder Metallschichten. Die meisten dieser Pigmente basieren auf plättchenförmigen Substraten aus Metallen oder natürlichen Schichtsilikaten, wie Glimmer, Talk oder Glas.

Dabei weisen insbesondere die Schichtsilikate den Nachteil auf, dass die Dicke des Substrates in einem weiten Bereich variiert und nicht gezielt einstellbar ist, was dazu führt, dass auch bei transparenten Substraten Lichttransmission und -reflexion am Substrat weitgehend unkontrollierbar ablaufen und daher nicht gezielt nutzbar sind.

Aus WO 93/08237 sind Effektpigmente auf der Basis von SiO₂-Plättchen bekannt, die sich mittels einer Bandtechnologie mit einer geringeren Schichtdickenvarianz herstellen lassen. Die Dicke der dort beschriebenen SiO₂-Plättchen beträgt üblicherweise zwischen 200 nm und 2 µm, insbesondere 500 nm.

Mit den bislang bekannten Pigmente lassen sich nicht alle gewünschten Effekte realisieren, so dass ein stetiger Bedarf an neuen Pigmenten besteht, die sich universell in verschiedensten Anwendungen einsetzen lassen, und neuartige und interessante Farbeffekte zeigen.

Die oben genannte Aufgabe wird durch Pigmente gemäß der vorliegenden Erfindung gelöst. Gegenstand der vorliegenden Erfindung sind demnach Effektpigmente mit verbesserten optischen Eigenschaften auf der Basis von mit einer oder mehreren Schichten beschichteten SiO₂-Plättchen, wobei die SiO₂-Plättchen eine Dicke von 50 nm bis 150 nm aufweisen.

Ebenfalls Gegenstand der vorliegenden Erfindung sind Verfahren zur Herstellung dieser Pigmente, bei denen SiO₂-Plättchen mit einer Dicke von 50 nm bis 150 nm mit einer oder mehreren Schichten beschichtet werden.

Die erfindungsgemäßen Pigmente haben den Vorteil, dass sie universell in den unterschiedlichsten Anwendungen eingesetzt werden können. Dabei zeigen die Effektpigmente neuartige Effekte, die mit den Pigmenten aus dem Stand der Technik nicht realisierbar bzw. in dieser Form nicht zu erwarten waren. Grundlage für diese unerwarteten Effekte ist die Kombination mehrerer Eigenschaften der erfindungsgemäßen Effektpigmente. Zum einen haben die Effektpigmente ein sehr dünnes Substrat mit einer Dicke im Bereich von 50 bis 150 nm, das in besonderer Weise die optischen Eigenschaften der Effektpigmente beeinflusst. Zum anderen handelt es sich bei dem Substrat um synthetische SiO₂-Plättchen, die sich durch hohe Transparenz und eine einheitliche Schichtdicke auszeichnen. Die Kombination dieser besonderen Merkmale führt zu überraschenden und einzigartigen Effekten bei entsprechenden Effektpigmenten und eröffnet somit den Zugang zu neuartigen Pigmenten mit besonderen Eigenschaften.

Aufgrund der vorteilhaften Eigenschaften eignen sich die erfindungsgemäßen Effektpigmente universell für eine große Anzahl unterschiedlichster Anwendungen. Gegenstand der vorliegenden Erfindung ist demgemäss auch die Verwendung dieser Pigmente in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten.

Die erfindungsgemäßen Pigmente basieren auf plättchenförmigen Siliziumdioxidpartikeln, welche über eine einheitliche Schichtdicke verfügen und vorzugsweise gemäß der internationalen Anmeldung WO 93/08237 auf einem endlosen Band durch Verfestigung und Hydrolyse einer Wasserglaslösung hergestellt werden. Unter einheitlicher Schichtdicke wird dabei eine Schichtdickentoleranz von 3 bis 10 %, bevorzugt von 3 bis 5 % der Gesamttrockenschichtdicke der Partikel verstanden. Die plättchenförmigen Siliziumdioxidpartikel liegen im allgemeinen in amorpher Form vor. Synthetische Plättchen dieser Art haben gegenüber natürlichen Materialien, wie z. B. Glimmer, den Vorteil, dass die Schichtdicke im Hinblick auf die gewünschten Effekte eingestellt werden kann und die Schichtdickentoleranz begrenzt ist.

Der Durchmesser der Substrate liegt üblicherweise zwischen 1 und 250 µm, vorzugsweise zwischen 2 und 100 µm. Ihre Dicke beträgt zwischen 50 und 150 nm. Das mittlere Aspektverhältnis der plättchenförmigen Substrate, das heißt das Verhältnis vom mittleren Längenmesswert, der hier dem mittleren Durchmesser entspricht, zum mittleren Dickenmesswert, beträgt üblicherweise 5 bis 200, bevorzugt 20 bis 150 und besonders bevorzugt 30 bis 120.

Die genannten Substrate sind in den erfindungsgemäßen Pigmenten mit einer oder mehreren Schichten beschichtet, vorzugsweise sind die SiO₂₋Plättchen mit einer Schicht beschichtet.

Die ein oder mehreren Schichten umfassen dabei Metalloxide, Metalloxidhydrate, Metallsuboxide, Metalle, Metallfluoride, Metallnitride, Metalloxynitride und/oder Mischungen dieser Materialien. Die Metalloxid-, Metalloxidhydrat-, Metallsuboxid-, Metall-, Metallfluorid-, Metallnitrid-, Metalloxynitridschichten und/oder die Mischungen hieraus können niedrig-(Brechzahl < 1,8) oder hochbrechend (Brechzahl ≥ 1,8) sein. Als Metalloxide und Metalloxidhydrate eignen sich alle dem Fachmann bekannten Metalloxide oder Metalloxidhydrate, wie z. B. Aluminiumoxid, Aluminiumoxidhydrat, Siliziumoxid, Siliziumoxidhydrat, Eisenoxid, Zinnoxid, Ceroxid, Zinkoxid, Zirkoniumoxid, Chromoxid, Titanoxid, insbesondere Titandioxid, Titanoxidhydrat sowie Mischungen hieraus, wie z. B. limenit oder Pseudobrookit. Als Metallsuboxide können beispielsweise die Titansuboxide eingesetzt werden. Als Metalle eignen sich z. B. Chrom, Aluminium, Nickel, Silber, Gold, Platin, Blei, Germanium, Titan, Kupfer oder Legierungen, als Metallfluorid eignet sich beispielsweise Magnesiumfluorid. Als Metallnitride oder Metalloxynitride können beispielsweise die Nitride oder Oxynitride der Metalle Titan, Zirkonium und/oder Tantal eingesetzt werden. Bevorzugt werden Metalloxid-, Metall-, Metallfluorid und/oder Metalloxidhydratschichten und ganz besonders bevorzugt Metalloxid-und/oder Metalloxidhydratschichten auf den SiO₂-Plättchen aufgebracht. Weiterhin können auch Mehrschichtaufbauten aus hoch- und niedrigbrechenden Metalloxid-, Metalloxidhydrat-, Metall- oder Metallfluoridschichten vorliegen, wobei sich vorzugsweise hoch- und niedrigbrechende Schichten abwechseln. Insbesondere bevorzugt sind Schichtpakete aus einer hoch- und einer niedrigbrechenden Schicht, wobei auf den SiO₂-Plättchen eines oder mehrere dieser Schichtpakete aufgebracht sein können. In einer weiteren Ausführungsform können die Metalloxid-, Metalloxidhydrat-, Metallsuboxid-, Metall-, Metallfluorid-, Metallnitrid-, Metalloxynitridschichten bzw. Mischungen hieraus mit Farbmitteln versetzt sein. Als Farbmittel eignen sich beispielsweise organische oder anorganische Farbpigmente wie farbige Metalloxide, z. B. Magnetit, Chromoxid oder Farbpigmente wie z. B. Berliner Blau, Ultramarin, Bismutvanadat, Thenards Blau, oder aber organische Farbpigmente wie z. B. Indigo, Azopigmente, Phthalocyanine oder auch Karminrot.

In einer bevorzugten Ausführungsform ist nur eine Schicht auf den SiO₂₋Plättchen aufgebracht. Diese eine Schicht umfasst vorzugsweise ein oder mehrere Metalloxide, insbesondere Titandioxid und/oder Eisenoxid.

Die Dicke der ein oder mehreren Schichten beträgt 20 bis 500 nm und wird idealerweise an den gewünschten Effekt bzw. an die Dicke der SiO₂₋Plättchen angepasst. So werden bei Beschichtung der SiO₂-Plättchen mit einer Schicht aus Titandioxid silberweiße Effektpigmente erhalten. Die Schichtdicke der SiO₂-Plättchen beträgt hierbei 50 bis 150 nm, vorzugsweise 50 bis 120 nm und ganz besonders bevorzugt 50 bis 100 nm. Die Dicke der Titanoxidschicht liegt im Bereich von 20 bis 200 nm, insbesondere im Bereich von 30 bis 180 und ganz besonders bevorzugt im Bereich von 40 bis 150 nm. Nur durch den Einsatz der dünnen SiO₂₋Plättchen lassen sich silberweiße Effektpigmente realisieren, da bei größeren Substratdicken die dann auftretende Eigeninterferenz der Substratplättchen das Auftreten des silberweißen Effektes verhindert. Die erfindungsgemäßen silberweißen Effektpigmente zeichnen sich durch einen besonders reinen silberweißen Effekt und einen höheren Glanz aus, der so mit Pigmenten auf Basis von natürlichem Glimmer nicht erreicht werden kann.

Werden SiO₂-Plättchen mit einer Schichtdicke von 120 bis 150 nm mit einer oder mehreren Schichten, umfassend Titandioxid oder Eisenoxid, beschichtet, so werden farbige Interferenzpigmente erhalten. Die Schichtdicke der ein oder mehreren Schichten betragen hierbei üblicherweise 50 bis 500 nm, vorzugsweise 50 bis 400 nm und insbesondere 50 bis 250 nm. Die farbigen Interferenzpigmente weisen klarere Interferenzfarben als herkömmliche Interferenzpigmente auf, wobei die Interferenzfarben praktisch keine Winkelabhängigkeit zeigen. Bei Pigmenten aus dem Stand der Technik mit dickeren SiO₂-Substraten werden dagegen häufig winkelabhängige Farbeffekte beobachtet, die bei einer Reihe von Anwendungen und Farbgestaltungen nicht immer erwünscht sind.

Darüber hinaus eignen sich die erfindungsgemäßen Effektpigmente auch zur Bereitstellung von Effektpigmenten mit Non-Flake-Appearance. Bei der Non-Flake-Appearance handelt es sich um einen Effekt, der bei Einsatz der Pigmente im Anwendungsmedium, z. B. einem Lack, beobachtet werden kann. Bei Auftreten dieses Effektes kann das Auge die parallel orientierten Einzelpigmentpartikel im Anwendungsmedium nicht mehr auflösen, das heißt, die einzelnen Partikel werden vom Auge nicht mehr wahrgenommen. Dies ist insbesondere bei solchen Anwendungen wünschenswert, bei denen ein möglichst einheitlicher Gesamteindruck vermittelt werden soll, wie z. B. bei Automobil- oder Industrielacken. Die Erzielung dieses Effektes ist mit Pigmenten aus dem Stand der Technik kaum, größtenteils aber gar nicht realisierbar. So ist zwar beispielsweise BiOCl geeignet, diesen Effekt zu erzeugen, findet aber wegen der Instabilität gegenüber Licht nur geringe Anwendung. Die erfindungsgemäßen Effektpigmente erlauben also die Bereitstellung von Pigmenten mit Non-Flake-Appearance bei gleichzeitiger Sicherstellung einer ausreichenden Stabilität der Pigmente gegenüber Licht und chemischen Einflüssen. Erfindungsgemäße Effektpigmente mit Non-Flake-Appearance basieren auf SiO₂-Plättchen mit einer Schichtdicke von 50 bis 100 nm, die mit einer oder mehreren Schichten beschichtet sind, wobei vorzugsweise eine Schicht eines Metalloxids, insbesondere aus Titandioxid und/oder Eisenoxid, vorliegt. Die Schichtdicke beträgt dabei 50 bis 500 nm, vorzugsweise 20 bis 200 nm.

Auf der Basis der dünnen SiO₂-Plättchen lassen sich also Effektpigmente mit unterschiedlichen Farbgebungen und Effekten erzeugen, die auf anderem Wege nur schwer oder gar nicht realisierbar sind.

In einer weiteren Ausführungsform der vorliegenden Erfindung können die erfindungsgemäßen Effektpigmente weiterhin mit einer zusätzlichen stabilisierenden organischen und/oder anorganischen Beschichtung als äußere Schicht versehen sein. Beispiele für derartige Beschichtungen finden sich z. B. in EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 oder EP 0 465 805, deren Offenbarung hiermit unter Bezugnahme mit eingeschlossen ist. Effektpigmente enthaltend eine organische Beschichtung, z. B. aus Organosilanen oder Organotitanaten bzw. Organozirkonaten zeigen neben den bereits genannten verbesserten optischen Eigenschaften zusätzlich eine erhöhte Stabilität gegenüber Witterungseinflüssen, wie z. B. Feuchtigkeit und Licht, was vor allem für Industrielacke und im Automobilbereich von besonderem Interesse ist. Die Stabilisierung kann durch anorganische Komponenten der zusätzlichen Beschichtung verbessert werden. Insgesamt sind die jeweiligen Anteile für die zusätzliche stabilisierende Beschichtung so auszuwählen, dass die optischen Eigenschaften der erfindungsgemäßen Effektpigmente nicht wesentlich beeinflusst werden.

Als Organosilane für die zusätzliche stabilisierende Beschichtung eignen sich beispielsweise Silane der allgemeinen Formel

X₄₋ₙ₋ₘZ-Rₙ(-B-Y)ₘ

mit X = OH, Halogen, Alkoxy, Aryloxy
Z=Si
R = Alkyl, Phenyl oder Wasserstoff
B = organische, zumindest bifunktionelle Gruppe (Alkylen, Alkylenoxyalkylen)
Y = Alkyl-, Amino-, substituierte Amino-, Hydroxy-, Hydroxyalkyl-, Siloxan-, Acetoxy, Isocyanat-, Vinyl-, Acryloyl-, Epoxy-, Epoxypropyloxy-, Imidazol- oder Ureidogruppe
n, m = 0,1,2,3 mit n+m ≤ 3 bestehen.

Die Organosilane bestehen aus einer Ankergruppe (X₄₋ₙ₋ₘZ), die z. B. an die Oberfläche des Pigmentes binden kann, wenigstens einer hydrophoben Gruppe (R,B) sowie einer oder mehrerer Alkyl- bzw. funktioneller Gruppen (Y). Bevorzugt besteht die Ankergruppe aus Alkoxysilanen, die durch hydrolytische Reaktionsbedingungen in entsprechende Hydroxygruppen überführt werden können.

Durch die Wahl geeigneter funktioneller Gruppen kann das Organosilan den Anforderungen angepasst werden. Darüber hinaus können, je nach Beschichtungsreihenfolge, durch Reaktion der funktionellen Gruppen mit entsprechenden Funktionalitäten in den Applikationsmedien zusätzliche Bindungen zwischen Pigment und Medium über das Organosilan erzeugt werden. In einer besonderen Ausführungsform wird die Oberfläche der erfindungsgemäßen Effektpigmente mit einer dem Einsatzmedium angepassten Kombination von organischen Funktionalitäten modifiziert. Hierzu eignet sich auch der Einsatz von Mischungen verschiedener Organosilane. Die Hydrophobie der Partikeloberfläche kann durch Integration von alkylhaltigen Kupplungsreagenzien, wie z. B. Alkylsilanen, ebenfalls angepasst werden. Neben den Organosilanen ist auch der Einsatz ihrer Hydrolysate sowie ihrer homogenen und heterogenen Oligomere und/oder Polymere bevorzugt, die ebenfalls alleinig oder in Kombination mit den bereits beschriebenen Silanen eingesetzt werden können. Im besonderen bevorzugt sind Mischungen verschiedener Organosilane, insbesondere mit voneinander unterschiedlichen funktionellen Gruppen Y, deren Einsatz eine besondere Anwendungsbreite gewährleistet.

Beispiele für Organosilane sind Propyltrimethoxysilan, Propyltriethoxysilan, Isobutyltrimethoxysilan, n-Octyltrimethoxysilan, i-Octyltrimethoxysilan, n-Octyltriethoxysilan, n-Decyltrimethoxysilan, Dodecyltrimethoxysilan, Hexadecyltrimethoxysilan, Vinyltrimethoxysilan, Octadecyltrimethoxysilan vorzugsweise Vinyltrimethoxysilan. Als oligomere, alkoholfreie Organosilanhydrolysate eignen sich unter anderem die unter dem Handelsnamen Dynasylan®" von der Fa. Sivento vertriebenen Produkte, wie z. B. Dynasylan HS 2926, Dynasylan HS 2909, Dynasylan HS2907, Dynasylan HS 2781, Dynasylan HS 2776, Dynasylan HS 2627. Darüber hinaus eignet sich oligomeres Vinylsilan als auch Aminosilanhydrolysat als organische Beschichtung. Funktionalisierte Organosilane sind beispielsweise 3-Aminopropyltrimethoxysilan, 3-Methacryloxytrimethoxysilan, 3-Glycidyloxypropyltrimethoxysilan, beta-(3,4-Epoxycyclohexyl)-ethyltrimethoxysifan, gamma-Isocyanatopropyltrimethoxysilan, 1,3-bis(3-glycidoxypropyl)-1,1,3,3,-tetramethyldisiloxan, Ureidopropyltriethoxysilan, bevorzugt sind 3-Aminopropyltrimethoxysilan, 3-Methacryloxytrimethoxysilan, 3-Glycidyloxypropyltrimethoxysilan, beta-(3,4-Epoxycyclohexyl)-ethyltrimethoxysilan, gamma-Isocyanatopropyltrimethoxysilan. Beispiele für polymere Silansysteme sind in WO 98/13426 beschrieben und werden z. B. von der Fa. Sivento unter dem Warenzeichen Hydrosil® vertrieben.

Ebenfalls Gegenstand der vorliegenden Erfindung sind Verfahren zur Herstellung der erfindungsgemäßen Effektpigmente, bei denen SiO₂₋Plättchen mit einer Dicke von 50 nm bis 150 nm mit einer oder mehreren Schichten beschichtet werden. Geeignete Materialien für die ein oder mehreren Schichten sind bereits vorab genannt.

Die Beschichtung mit einer oder mehreren Schichten kann nasschemisch, mittels Sol-Gel-Verfahren und/oder durch CVD- oder PVD-Verfahren erfolgen.

Vorzugsweise handelt es sich bei den erfindungsgemäßen Verfahren zur Herstellung der Effektpigmente um nasschemische Verfahren, bei denen die bekannten, zur Herstellung von Perlglanzpigmenten entwickelten nasschemischen Beschichtungstechnologien angewendet werden können, die beispielsweise in den folgenden Publikationen beschrieben sind:
DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017.

Zur Beschichtung wird das plättchenförmige Substrat in Wasser suspendiert und ein- oder mehrfach mit einem Metalloxid, Metalloxidhydrat, Metallsuboxid, Metall, Metallfluorid, Metallnitrid, Metalloxynitrid und/oder Mischungen hieraus durch Zugabe und Fällung der entsprechenden anorganischen Metallverbindungen beschichtet, wobei durch gleichzeitige Zugabe von Säure oder Base der für die Fällung des jeweiligen Metalloxids, Metalloxidhydrats, Metallsuboxids, Metalls, Metallfluorids, Metallnitrids, Metalloxynitrids notwendige pH-Wert eingestellt und konstant gehalten wird und anschließend das beschichtete Substrat aus der wässrigen Suspension abgetrennt, getrocknet und gegebenenfalls kalziniert wird.

Die Kalzinierungstemperatur kann dabei im Hinblick auf die jeweils vorhandene Beschichtung optimiert werden. Im Allgemeinen liegt die Kalzinierungstemperatur jedoch zwischen 250 und 1000 °C, insbesondere zwischen 350 und 900 °C. Die Pigmente können auch im Falle der Aufbringung mehrerer Schichten nach dem Aufbringen jeder einzelnen Schicht abgetrennt, getrocknet und gegebenenfalls kalziniert werden, bevor sie zur Aufbringung der nächsten Schicht erneut dispergiert werden.

Wenn die Schicht TiO₂ umfasst, wird zum Aufbringen dieser Schichten bevorzugt das in US 3,553,001 beschriebene Verfahren eingesetzt. Dabei wird zu einer auf etwa 50 - 100 °C, insbesondere 70 - 80 °C erhitzten Suspension der plättchenförmigen, gegebenenfalls bereits vorbeschichteten, Substrate langsam eine wässrige Lösung eines anorganischen Titansalzes zugegeben und der pH-Wert durch gleichzeitiges Zudosieren einer Base bei 0,5 bis 5, insbesondere etwa 1,5 bis 2,5, weitgehend konstant gehalten. Sobald die gewünschte Schichtdicke des TiO₂-Oxidhydrates erreicht ist, wird die Zugabe der Titansalzlösung und der Base gestoppt. Dieses Verfahren wird auch als Titrationsverfahren bezeichnet und weist die Besonderheit auf, dass kein Überschuss an Titansalz vorliegt, sondern pro Zeiteinheit immer nur eine solche Menge bereitgestellt wird, wie für eine gleichmäßige Beschichtung mit dem hydratisierten TiO₂ erforderlich ist und auch von der Oberfläche des zu beschichtenden Substrates aufgenommen werden kann. In der Lösung sind daher keine hydratisierten Titandioxidteilchen vorhanden, die nicht auf der zu beschichtenden Oberfläche abgeschieden werden.

Prinzipiell sind für die Herstellung der erfindungsgemäßen Pigmente auch CVD- oder PVD-Verfahren zur Beschichtung, insbesondere mit Metallen, geeignet. Dabei ist es erforderlich, dass das Substrat während des Bedampfungsvorganges gleichmäßig in Bewegung gehalten wird, damit eine homogene Beschichtung aller Oberflächen gewährleistet ist.

Darüber hinaus kann in einem ebenfalls erfindungsgemäßen Verfahren zusätzlich als äußere Schicht eine stabilisierende organische und/oder anorganische Beschichtung aufgebracht werden. Beispiele für derartige Beschichtungsverfahren finden sich unter anderem in EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 oder EP 0 465 805. Beispiele für organische Beschichtungen sowie die damit verbundenen Vorteile sind bereits vorab beim Aufbau der erfindungsgemäßen Pigmente beschrieben worden. Der Verfahrensschritt der Aufbringung der organischen Beschichtung kann direkt an die anderen Schritte des erfindungsgemäßen Verfahrens angeschlossen werden. Die hierbei aufgebrachten Stoffe umfassen lediglich einen Gewichtsanteil von 0,1 bis 5 Gew.-%, vorzugsweise 0,5 bis 3 Gew.-%, des gesamten Pigmentes.

Die erfindungsgemäßen Effektpigmente sind vielseitig einsetzbar und können in vielen Bereichen eingesetzt werden. Demgemäss ist die Verwendung der erfindungsgemäßen Pigmente in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten ebenfalls Gegenstand der vorliegenden Erfindung.

Im Falle von Kosmetika eignen sich die erfindungsgemäßen Effektpigmente besonders für Produkte und Formulierungen der dekorativen Kosmetik, wie z. B. Nagellacke, farbgebende Puder, Lippenstifte oder Lidschatten, Seifen, Zahnpasten etc. Selbstverständlich können die erfindungsgemäßen Effektpigmente in den Formulierungen auch mit jeder Art von kosmetischen Roh- und Hilfsstoffen kombiniert werden. Dazu gehören u. a. Öle, Fette, Wachse, Filmbildner, Konservierungsmittel und allgemein anwendungstechnische Eigenschaften bestimmende Hilfsstoffe, wie z. B. Verdicker und rheologische Zusatzstoffe wie etwa Bentonite, Hektorite, Siliziumdioxid, Ca-Silikate, Gelatine, hochmolekulare Kohlenhydrate und/oder oberflächenaktive Hilfsmittel, etc. Die erfindungsgemäßen Effektpigmente enthaltenden Formulierungen können dem lipophilen, hydrophilen oder hydrophoben Typ angehören. Bei heterogenen Formulierungen mit diskreten wässrigen und nicht-wässrigen Phasen können die erfindungsgemäßen Partikel in jeweils nur einer der beiden Phasen enthalten oder auch über beide Phasen verteilt sein.

Die pH-Werte der wässrigen Formulierungen können zwischen 1 und 14, bevorzugt zwischen 2 und 11 und besonders bevorzugt zwischen 5 und 8 liegen. Den Konzentrationen der erfindungsgemäßen Effektpigmente in der Formulierung sind keine Grenzen gesetzt. Sie können - je nach Anwendungsfall - zwischen 0.001 (rinse-off-Produkte, z. B. Duschgele) und 99 % (z. B. Glanzeffekt-Artikel für besondere Anwendungen) liegen. Die erfindungsgemäßen Effektpigmente können weiterhin auch mit kosmetischen Wirkstoffen kombiniert werden. Geeignete Wirkstoffe sind z. B. Insect Repellents, UV A/BC-Schutzfilter (z. B. OMC, B3, MBC), Anti-Ageing-Wirkstoffe, Vitamine und deren Derivate (z. B. Vitamin A, C, E etc.), Selbstbräuner (z. B. DHA, Erythrolose u.a.) sowie weitere kosmetische Wirkstoffe wie z. B. Bisabolol, LPO, Ectoin, Emblica, Allantoin, Bioflavanoide und deren Derivate.

Bei Einsatz der Effektpigmente in Lacken und Farben sind alle dem Fachmann bekannten Anwendungsbereiche möglich, wie z. B. Pulverlacke, Automobillacke, Druckfarben für den Tief-, Offset-, Sieb- oder Flexodruck sowie für Lacke in Außenanwendungen. Die Lacke und Farben können hierbei beispielsweise strahlungshärtend, physikalisch trocknend oder chemisch härtend sein. Für die Herstellung der Druckfarben oder Flüssiglacke ist eine Vielzahl von Bindern, z.B. auf der Basis von Acrylaten, Methacrylaten, Polyestern, Polyurethanen, Nitrocellulose, Ethylcellulose, Polyamid, Polyvinylbutyrat, Phenolharzen, Maleinharzen, Stärke oder Polyvinylalkohol, Aminharzen, Alkydharzen, Epoxidharzen, Polytetrafluorethylen, Polyvinylidenfluoriden, Polyvinylchlorid oder Mischungen hieraus geeignet, insbesondere wasserlösliche Typen. Bei den Lacken kann es sich um Pulverlacke oder wasser- oder lösemittelbasierte Lacke handeln, wobei die Auswahl der Lackbestandteile dem Allgemeinwissen des Fachmanns unterliegt. Gängige polymere Bindemittel für Pulverlacke sind beispielsweise Polyester, Epoxide, Polyurethane, Acrylate oder Mischungen hieraus.

Darüber hinaus können die erfindungsgemäßen Effektpigmente in Folien und Kunststoffen verwendet werden, so z. B. in Agrarfolien, infrarotreflektierenden Folien und Scheiben, Geschenkfolien, Kunststoffbehältnissen und Formkörpern für alle dem Fachmann bekannten Anwendungen. Als Kunststoffe eignen sich alle gängigen Kunststoffe für die Einarbeitung der erfindungsgemäßen Effektpigmente, z. B. Duromere oder thermoplastische Kunststoffe. Die Beschreibung der Anwendungsmöglichkeiten und der einsetzbaren Kunststoffe, Verarbeitungsverfahren und Additive finden sich z. B. in der RD 472005 oder in R. Glausch, M. Kieser, R. Maisch, G. Pfaff, J. Weitzel, Perlglanzpigmente, Curt R. Vincentz Verlag, 1996, 83 ff., deren Offenbarungsgehalt hier mit umfasst ist.

Darüber hinaus eignen sich die erfindungsgemäßen Effektpigmente auch für den Einsatz im Sicherheitsdruck und in sicherheitsrelevanten Merkmalen für z. B. fälschungssichere Karten und Ausweise, wie z. B. Eintrittskarten, Personalausweise, Geldscheine, Schecks und Scheckkarten sowie für andere fälschungssichere Dokumente. Im Bereich der Landwirtschaft können die Effektpigmente zur Einfärbung von Saatgut und anderen Ausgangsgütern verwendet werden, darüber hinaus im Lebensmittelbereich zur Pigmentierung von Lebensmitteln. Zur Pigmentierung von Überzügen in Arzneimitteln wie z. B. Tabletten oder Dragees sind die erfindungsgemäßen Effektpigmente ebenfalls einsetzbar.

Die erfindungsgemäßen Effektpigmente eignen sich in den oben genannten Anwendungsgebieten ebenso zur Verwendung in Abmischungen mit organischen Farbstoffen und/oder Pigmenten, wie z. B. transparenten und deckenden Weiß-, Bunt- und Schwarzpigmenten, lumineszierenden Farbstoffen und/oder Pigmenten sowie mit plättchenförmigen Eisenoxiden, organischen Pigmenten, holographischen Pigmenten, LCPs (Liquid Crystal Polymers) und herkömmlichen transparenten, bunten und schwarzen Glanzpigmenten auf der Basis von metalloxidbeschichteten Plättchen auf Basis von Glimmer, Glas, Al₂O₃, Fe₂O₃, SiO₂, etc. Die erfindungsgemäßen Effektpigmente können in jedem Verhältnis mit handelsüblichen Pigmenten und Füllern gemischt werden.

Als Füllstoffe sind z. B. natürlicher und synthetischer Glimmer, Nylon Powder, reine oder gefüllte Melaninharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe zu nennen. Bezüglich der Partikelform des Füllstoffes gibt es keine Einschränkungen. Sie kann den Anforderungen gemäß z. B. plättchenförmig, sphärisch oder nadelförmig sein.

Die erfindungsgemäßen Effektpigmente sind weiterhin geeignet zur Herstellung von fließfähigen Pigmentpräparationen und Trockenpräparaten enthaltend ein oder mehrere erfindungsgemäße Partikel, Bindemittel und optional ein oder mehrere Additive. Unter Trockenpräparate sind auch Präparate zu verstehen, die 0 bis 8 Gew.-%, vorzugsweise 2 bis 8 Gew.-%, insbesondere 3 bis 6 Gew.-%, an Wasser und/oder eines Lösemittels oder Lösemittelgemisches enthalten. Die Trockenpräparate liegen vorzugsweise als Pellets, Granulate, Chips, Würstchen oder Briketts vor und weisen Teilchengrößen von 0,2 - 80 mm auf. Die Trockenpräparate finden insbesondere Anwendung bei der Herstellung von Druckfarben und in kosmetischen Formulierungen.

Die vollständige Offenbarung aller vorstehend genannten Patentanmeldungen, Patente und Veröffentlichungen ist durch Bezugnahme in dieser Anmeldung enthalten.

Die nachfolgenden Beispiele sollen die Erfindung näher erläutern, ohne sie jedoch zu begrenzen.

### Beispiele

### Beispiel 1:

100 g SiO₂-Plättchen (125 nm dick, Korngröße 5 - 50 µm) werden in 2 l vollentsalztem Wasser auf 75 °C erhitzt. Bei einer Rührgeschwindigkeit von 1300 Upm werden 17 g 50 %-ige SnCl₄ Lösung zugegeben. Dabei wird der pH-Wert mit Kalilauge (30 Gew.-%) konstant bei pH 1,4 gehalten. Anschließend wird der pH-Wert mit Salzsäure (10 Gew.-%) auf pH 1,3 gesenkt. Bei diesem pH-Wert werden 600 g TiCl₄-Lösung (400 g/l) zugegeben. Der pH-Wert von 1,3 wird mit Kalilauge (30 %) konstant gehalten. Nach Zugabe der TiCl₄-Lösung wird der pH-Wert mit Kalilauge (30 %) auf pH 6 angehoben und 15 min nachgerührt. Das Produkt wird abfiltriert und mit vollentsalztem Wasser nachgewaschen. Nach Trocknung bei 110 °C wird es bei 800 °C kalziniert.

Man erhält ein silberweißes Effektpigment mit hohem Glanz.

### Beispiel 2:

100 g SiO₂-Plättchen (125 nm dick, Korngröße 5 - 50 µm) werden in 2 I vollentsalztem Wasser auf 75 °C erhitzt. Bei einer Rührgeschwindigkeit von 1300 Upm werden 17 g 50 %-ige SnCl₄ Lösung zugegeben. Dabei wird der pH-Wert mit Kalilauge (30 Gew.-%) konstant bei pH 1,4 gehalten. Anschließend wird der pH-Wert mit Salzsäure (10 Gew.-%) auf pH 1,3 gesenkt. Bei diesem pH-Wert werden 1200 g TiCl₄-Lösung (400 g/l) zugegeben. Der pH-Wert von 1,3 wird mit Kalilauge (30 %) konstant gehalten. Nach Zugabe der TiCl₄-Lösung wird der pH-Wert mit Kalilauge (30 %) auf pH 6 angehoben und 15 min nachgerührt. Das Produkt wird abfiltriert und mit vollentsalztem Wasser nachgewaschen. Nach Trocknung bei 110 °C wird es bei 800 °C kalziniert.

Man erhält ein Interferenzpigment mit hohem Glanz und blauer Interferenzfarbe.

### Beispiel 3:

100 g SiO₂-Plättchen (80 nm dick, Korngröße 5 - 50 µm) werden in 2 l vollentsalztem Wasser auf 75 °C erhitzt. Bei einer Rührgeschwindigkeit von 1300 Upm werden 17 g 50%-ige SnCl₄ Lösung zugegeben. Dabei wird der pH-Wert mit Kalilauge (30 Gew.-%) konstant bei pH 1,4 gehalten. Anschließend wird der pH-Wert mit Salzsäure (10 Gew.-%) auf pH 1,3 gesenkt. Bei diesem pH-Wert werden 1200 g TiCl₄-Lösung (400 g/l) zugegeben. Der pH-Wert von 1.3 wird mit Kalilauge (30 %) konstant gehalten. Nach Zugabe der TiCl₄-Lösung wird der pH-Wert mit Kalilauge (30 %) auf pH 6 angehoben und 15 min nachgerührt. Das Produkt wird abfiltriert und mit vollentsalztem Wasser nachgewaschen. Nach Trocknung bei 110 °C wird es bei 800 °C kalziniert.

Man erhält ein Effektpigment, das nach Einarbeitung in ein Anwendungsmedium Non-Flake-Appearance zeigt, das heißt die einzelnen Pigmentpartikel können vom Auge nicht aufgelöst werden.

## Patentansprüche

1. Effektpigmente mit verbesserten optischen Eigenschaften auf der Basis von mit einer oder mehreren Schichten beschichteten SiO₂-Plättchen, **dadurch gekennzeichnet, dass** die SiO₂-Plättchen eine Dicke von 50 nm bis 150 nm aufweisen.

2. Effektpigmente gemäß Anspruch 1, **dadurch gekennzeichnet, dass** die SiO₂-Plättchen mit einer Schicht beschichtet sind.

3. Effektpigmente gemäß Anspruch 1 oder 2, **dadurch gekennzeichnet, dass** die ein oder mehreren Schichten Metalloxide, Metalloxidhydrate, Metallsuboxide, Metalle, Metallfluoride, Metallnitride, Metalloxynitride und/oder Mischungen dieser Materialien umfassen.

4. Effektpigmente gemäß Anspruch 3, **dadurch gekennzeichnet, dass** das Metalloxid Eisenoxid und/oder Titandioxid ist.

5. Effektpigmente gemäß einem der Ansprüche 1 bis 4, **dadurch gekennzeichnet, dass** die Dicke der ein oder mehreren Schichten 20 - 500 nm beträgt.

6. Effektpigmente gemäß einem der Ansprüche 1 bis 5, **dadurch gekennzeichnet, dass** es sich bei den Effektpigmenten um silberweiße Effektpigmente, farbige Interferenzpigmente oder Effektpigmente mit Non-Flake-Appearance handelt.

7. Effektpigmente gemäß einem der Ansprüche 1 bis 6, **dadurch gekennzeichnet, dass** die Effektpigmente weiterhin mit einer zusätzlichen organischen Beschichtung als äußere Schicht versehen sind.

8. Verfahren zur Herstellung von Effektpigmenten gemäß Anspruch 1, **dadurch gekennzeichnet, dass** SiO₂-Plättche mit einer Dicke von 50 nm bis 150 nm mit einer oder mehreren Schichten beschichtet werden.

9. Verfahren gemäß Anspruch 8, **dadurch gekennzeichnet, dass** die Beschichtung mit einer oder mehreren Schichten nasschemisch, durch Sol-Gel-Verfahren und/oder durch CVD- oder PVD-Verfahren erfolgt.

10. Verwendung von Effektpigmenten gemäß Anspruch 1 in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten.
